(19) Europäisches Patentamt
European Patent Office
Office européen des brevets

(11) Veröffentlichungsnummer: **0 192 135**
**A2**

(12) # EUROPÄISCHE PATENTANMELDUNG

(21) Anmeldenummer: 86101588.1

(22) Anmeldetag: 07.02.86

(51) Int. Cl.[4]: C 07 K 5/08
A 61 K 37/02

(30) Priorität: 18.02.85 DE 3505555

(43) Veröffentlichungstag der Anmeldung:
27.08.86 Patentblatt 86/35

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Stüber, Werner, Dr.
Cölber Weg 12
D-3551 Lahntal(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Neue Oligopeptidylargininolderivate und deren Homologe, Verfahren zu deren Herstellung, deren Verwendung und diese enthaltende Mittel.**

(57) Die Erfindung betrifft neue Peptidylargininolderivate und deren Homologe mit der allgemeinen Formel

X-D-Phe-Pro-A-Y.(HB)$_n$,

wobei

X ein Wasserstoffatom oder eine in der Peptidchemie übliche, bekannte Schutzgruppe ist,

D-Phe D-Phenylalanin ist,

Pro L-Prolin ist,

A ein ω-Guanidino-β-aminoalkanolrest der Formel $-NHCH[(CH_2)_mNHC(NH)NH_2]CH_2O-$ mit m = 2 bis 5 C-Atomen,

vorzugsweise ein Argininolrest oder ein Homoargininolrest ist,

Y ein Wasserstoffatom oder eine Ester-bildende Gruppe ist,

B ein Säurerest ist

n 0, 1 oder 2 ist,

Verfahren zur Herstellung dieser Verbindungen, pharmazeutische Mittel, die diese Mittel enthalten sowie die Verwendung dieser Mittel als Thrombininhibitoren.

EP 0 192 135 A2

Croydon Printing Company Ltd.

Neue Oligopeptidylargininolderivate und deren Homologe, Verfahren zu deren Herstellung, deren Verwendung und diese enthaltende Mittel

---

Die Erfindung betrifft neue Oligopeptidylargininolderivate und deren Homologe, die Synthese dieser Verbindungen, deren Verwendung sowie pharmazeutische Mittel, die die neuen, als Thrombin-inhibitoren hoch wirksamen Verbindungen enthalten.

Es ist bekannt, daß es unter einer ganzen Reihe von pathophysiologischen Bedingungen zur Bildung von Thrombin-Antithrombin (AT) III-Komplexen und damit zum Verbrauch des Proteaseinhibitors AT III, dem wichtigsten Thrombininhibitor im Humanplasma, kommt. Ein Abfall von AT III führt zu einem hohen Thromboserisiko, wie man u.a. auch von Fällen mit angeborenem Mangel an AT III weiß. Verminderungen auf Werte unterhalb 75 % der Norm haben thromboembolische Komplikationen zur Folge. Heute erfolgt die Therapie von angeborenen und erworbenen AT III-Mangelzuständen durch Zuführung von AT III, das aus dem Blutplasma freiwilliger Spender gewonnen wird. Da Blutplasma nur in begrenzten Mengen verfügbar ist, sind der klinischen Anwendung von AT III Grenzen gesetzt. Unter diesem Aspekt ist es wünschenswert, nach synthetischen Inhibitoren zu suchen, die vergleichbar dem natürlichen AT III die Enzymtasche des Thrombins verschließen oder auch den aktiven Serinrest der Protease Thrombin chemisch zu blockieren vermögen. Von der Spezifität des Thrombins her kommen dafür Derivate des Arginins in Betracht, weil sie gut in die Enzymtasche des Thrombins passen, und speziell solche, die als reaktive Gruppe

Aldehyde oder auch Chloromethyl-Ketone besitzen.

Es ist bekannt, daß Peptide auf der Basis Phe-Pro-Arg sich als günstig erweisen, besonders dann, wenn die N-terminale Aminosäure in der D-Form vorliegt (C. Mattson, E. Eriksson und S. Nilsson, Folia Haematol. 109, 43-51, 1982). Als kompetitiv wirkende Inhibitoren sind hier zu nennen: D-Phe-Pro-Arg-isopropylester, D-Phe-Pro-Arg-4-methylpiperidin.

Es ist ferner bekannt, daß sich eine Verstärkung der Inhibitorwirkung durch eine derartige Derivatisierung am C-Terminus durchführen läßt, daß nunmehr eine Reaktion mit dem "aktiven" Serin bzw. dem aktivierend wirkenden Histidin eintritt. Als besonders wirksam haben sich hier Argininale bzw. Chloromethylketone des Arginins erwiesen. Argininale sollten dabei in der Lage sein, das Serinhydroxyl halbacetalisch zu binden. Chloromethylketone hingegen sollten $N^{im}$-Alkylierungen bewirken. Bei beiden Inhibitormodellen bewirkte N-terminales D-Phe-Pro eine hohe Affinität zu Thrombin.

Bekannt sind eine Anzahl weiterer Verbindungen, die als Thrombininhibitoren wirken und die strukturell dem Arginin verwandt sind und Variationen am N- und C-Terminus zeigen. Erwähnt sei beispielsweise ε-Guanidinocapronsäure-p-carboxyethylphenylester (M. Muramatu und S. Fujii, Biochim. Biophys. Acta 268, 221-224, 1972).

Es wurde nun überraschenderweise gefunden, daß die am C-Terminus vollständig reduzierte Form des Peptids D-Phe-Pro-Arg, das D-Phe-Pro-Argininol, eine kräftige Thrombininhibition bewirkte.

Die Erfindung betrifft somit neue Substanzen, die in der Lage sind, Thrombin zu inhibieren und folgende Struktur

aufweisen:

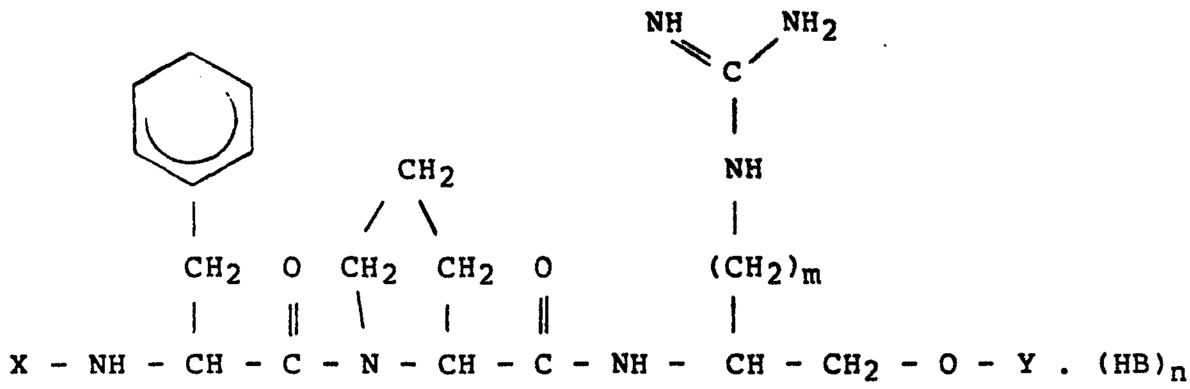

entsprechend der Schreibweise

X-D-Phe-Pro-A-Y·(HB)$_n$,

worin

X ein Wasserstoffatom oder eine in der Peptidchemie übliche Schutzgruppe ist, vorzugsweise Boc oder Z,

D-Phe D-Phenylalanin ist,

Pro L-Prolin ist,

A ein ω-Guanidino-ß-Aminoalkanol (Argininol und Homologe) der Formel $-NHCH[(CH_2)_mNHC(NH)NH_2]CH_2O-$, wobei m = 2 bis 5, vorzugsweise 3 oder 4 ist

Y ein Wasserstoffatom

oder

Y eine Ester-bildende Gruppierung der Struktur

OC-R mit R = H oder ein aliphatischer Rest mit

bis zu 4 Kohlenstoffatomen oder

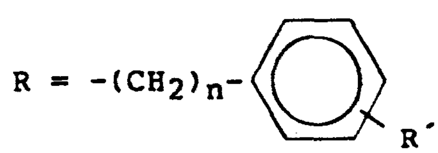

wobei $R' = H, CH_3, OCH_3, NO_2$
und $n = 0, 1$ oder $2$ ist,

oder

Y eine Ester-bildende Gruppierung der Struktur

$-SO_2-R$ mit $R = OH$, ein araliphatischer oder aromatischer Rest der Struktur

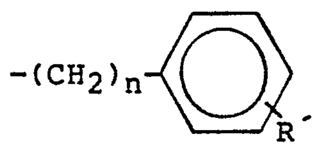

wobei $R' = H, CH_3, OCH_3$
und $n = 0, 1$ oder $2$ ist,

B ein Säurerest ist,
n 0, 1 oder 2 ist.

Die erfindungsgemäßen Verbindungen werden nach den in der Peptidchemie üblichen Methoden hergestellt, und zwar entweder durch Fragmentkondensation oder durch stufenweisen Aufbau, wobei temporär eingeführte Schutzgruppen abgespalten werden und gegebenenfalls die erhaltenen Verbindungen in ihre physiologisch verträglichen Salze überführt werden.

Zunächst wird das Argininol, das nachfolgend auch mit $ArgCH_2OH$ bezeichnet ist, oder dessen Homologes als Muttersubstanz synthetisiert. Hierzu arbeitet man beispielsweise entsprechend dem literaturbekannten Verfahren von E. Koltai et al. (E. Koltai, B. Horvath und D.

Banfi, J. Labelled Compds. 19, 7-11, 1982) für die Herstellung von L-$N^G$-nitroargininolhydrobromid, wobei auf eine $N^G$-Maskierung allerdings vorzugsweise verzichtet wird.

Das C-Atom 2 des Argininols bzw. das C-Atom 2 der Homologen des Argininols ist chiral, weshalb die D- und L-Form möglich sind.

Bevorzugt wird die L-Form eingesetzt.

Das Argininol bzw. Homoargininol sowie deren Homologe lassen sich durch Reduktion geeigneter Derivate mit komplexen Hydriden herstellen. Geeignete Derivate sind Alkylester mit 1-6 Kohlenstoffatomen; besonders geeignet sind die Methylester.

Um eine gute Löslichkeit zu garantieren und die Bildung von Nebenprodukten während der Reaktion zu vermeiden, werden die in der Peptidchemie üblichen Schutzgruppen eingesetzt. Als Schutzgruppen der primären Aminogruppe lassen sich zum Beispiel die Triphenylmethyl (Trt)-, Carbobenzoxy (Z)- oder Butyloxycarbonyl (Boc)-Gruppen verwenden. Bevorzugt wird die Trt-Gruppe eingesetzt. Als Reduktionsmittel verwendet man die aus dem Stand der Technik bekannten Verbindungen; vorzugsweise wird Lithiumaluminiumhydrid in absolutem Tetrahydrofuran oder Natriumborhydrid mit Calciumchlorid in Ethanol eingesetzt, besonders bevorzugt ist Lithiumaluminiumhydrid.

Die Reduktionen werden jeweils mit einem Überschuß an komplexen Hydriden durchgeführt. Die Produkte werden dünnschichtchromatographisch auf ihre Reinheit untersucht. Die Hydroxylfunktionen können infrarotspektroskopisch anhand der C-O-Schwingung bei 1040 $cm^{-1}$ erkannt

werden. Im Falle der Trt-Arg $CH_2OH$-Verbindung weist das Fehlen von Carbonylbanden im Infrarotspektrum eine vollständige Reduktion aus.

Die N -geschützten Argininole bzw. Homoargininole und Homologen lassen sich nach bekannten Methoden mit Säurechloriden oder Anhydriden in die entsprechenden Ester umsetzen, wobei vorzugsweise Säurechloride verwendet werden. Die N -Schutzgruppen werden von freien bzw. veresterten Argininolen oder Homoargininolen oder dem Homolog nach den bekannten Methoden abgespalten.

Die freien bzw. veresterten Argininole oder Homoargininole oder deren Homologe werden nach den in der Peptidchemie üblichen Arbeitsweisen mit dem Dipeptid D-Phe-Pro, das am N-Terminus vorzugsweise mit Boc oder Z geschützt ist, umgesetzt. Dafür sind Aktivesterkupplungen bevorzugt geeignet.

Im Zuge der Synthese der erfindungsgemäßen Verbindungen X-D-Phe-Pro-A-Y, entsprechend obengenannter Struktur, können selbstverständlich die in der Peptidchemie üblichen Schutzgruppen eingesetzt werden. Das heißt, die alkoholische Funktion von Argininol bzw. Homoargininol oder deren Homologen kann beispielsweise veräthert und die Guanidinogruppe beispielsweise als Nitroverbindung vorliegen. Das Dipeptidderivat kann sowohl als solches eingebaut werden, der Einbau kann aber auch stufenweise über den Einbau einzelner, geschützter, aktivierter Aminosäuren erfolgen. Als N-terminale Schutzgruppen kommen die in der Peptidchemie üblichen, vorzugsweise die physiologisch verträglichen Schutzgruppen in Frage, z. B. Butyloxycarbonyl-(Boc), Carbobenzoxy-(Z), Fluorenylmethyloxycarbonyl-(Fmoc), Biphenylylpropyloxycarbonyl-(Bpoc), vorzugsweise Boc oder Z.

Die Abspaltung der Schutzgruppen geschieht in bekannter Weise unter Verwendung der in der Peptidchemie hierfür üblicherweise benutzten Reagenzien.

Die vorzugsweise eingesetzte N -Schutzgruppe ist die Boc-Gruppe, diese wird vorzugsweise mit 1,2 molarem Chlorwasserstoff in Eisessig oder Trifluoressigsäure abgespalten.

Die erfindungsgemäßen Verbindungen zeigten überraschenderweise eine kräftige Inhibitorwirkung gegen Thrombin. Die antikoagulierende Wirkung ist sehr stark ausgeprägt bei den D-Phenylalanyl-L-propyl-L-argininol-Derivaten und den entsprechenden Homargininolderivaten, die Inhibitorwirkung ist ganz besonders stark bei dem D-Phenylalanyl-L-prolyl-L-argininol.
Daß die neuen Dipeptidylargininolderivate als Thrombininhibitoren besonders wirksam sind, ist vor allem auch deshalb überraschend, weil dieser Effekt im deutlichen Gegensatz zu Erkenntnissen steht, die an der Spermien-Endoprotease Akrosin gefunden wurden (G. Borin, G. Chessa, G. Cavaggion, F. Marchiori und W. Müller-Esterl, Hoppe-Seyler`s Z. Physiol. Chem. 362, 1435-1445, 1981). Dabei wurde gezielt Boc-Leu-Leu-Argininal . Essigsäure . $H_2O$ hergestellt und als hochwirksamer Inhibitor erkannt. Das in diesem Zusammenhang ebenfalls hergestellte entsprechende Argininolderivat wird als im Inhibitionstest vollständig inaktiv beschrieben. Hiernach war somit keineswegs zu erwarten, daß die Argininderivate der Erfindung ungewöhnlich wirksame Thrombin-Inhibitoren darstellen.

Die Verbindungen gemäß der Erfindung eignen sich als Ersatz für die natürlich vorkommenden Proteaseinhibitoren, vor allem für das Antithrombin III, das - wie eingangs erwähnt - aus dem Blut von Spendern gewonnen wird und schon deshalb nur begrenzt zur Verfügung steht. Dabei ist die Inhibitorwirkung der erfindungsgemäßen Verbindungen spezifischer als bei anderen bekannten Inhibitoren, z. B. bei dem ε-Guanidino-capronsäure-4-carboxyethylphenylester.

Die erfindungsgemäßen Substanzen und deren physiologisch verträglichen Salze können als wirksame Komponenten für Mittel dienen, durch die AT III-Mangel behoben und damit das Thromboserisiko eliminiert oder zumindest stark vermindert werden. Diese Mittel können zusätzlich physiologisch unbedenkliche Träger oder sonstige Hilfsstoffe enthalten. Bei parenteraler Applikation können z. B. auch Lösungsvermittler, Emulgatoren und dgl. mit deren Hilfe die erfindungsgemäßen Substanzen in Lösung, Suspension oder Emulsion gebracht werden. Als Lösungsmittel kommen in Frage: Wasser, physiologische Kochsalzlösungen oder Alkohole, z. B. Ethanol, Propanol oder Glycerin, daneben auch Zuckerlösungen wie Glucose oder Mannitlösungen oder auch eine Mischung von verschiedenen Lösungsmitteln, bevorzugt wird eine physiologische Kochsalzlösung eingesetzt. Für orale Applikation kommen die üblichen galenischen Zubereitungen in Frage.

Abkürzungen

$N^G$ - primäre Aminogruppe der Guanidinogruppe
Boc - tert. Butoxycarbonyl
Trt - Triphenylmethyl
Z - Benzyloxycarbonyl (= Carbobenzoxy)
$k_i$ - Inhibitorkonstante
$N^{im}$ - Stickstoffatom des Histidinimidazolringes
min - Minuten
DC - Dünnschichtchromatographie

| | |
|---|---|
| $R_F$ | - Retentionsfaktor |
| C/T | - Chlor/4,4 -Bis-(dimethylamino)diphenylmethan-Test |
| UV | - Ultraviolettvisualisation bei 254 nm |
| DMF | - Dimethylformamid |

## Beispiele

Laufmittel für die Dünnschichtchromatographie/Kieselgel-chromatographie:

| | |
|---|---|
| A: Butanol/Eisessig/Wasser | 3:1:1 |
| B: Chloroform/Methanol/Eisessig | 50:10:2,5 |
| C: Chloroform/Methanol/Eisessig | 50:10:5 |
| D: Chloroform/Methanol/Eisessig | 50:20:5 |
| E: Chloroform/Methanol/Eisessig/Wasser | 20:10:2:1 |

## Beispiel 1

### Stufe 1:

Tritylargininmethylesterhydrochlorid wurde nach Literatur (R. A. Boissonas, St. Guttmann, R. L. Huguenin, R.-A. Jaquenoud und E. Sandrin, Helv. Chim. Acta, 16, 1875, 1958) hergestellt und wie in Stufe 2 beschrieben zur Reduktion gebracht.

### Stufe 2: Tritylargininolhydrochlorid (Trt-Arg $CH_2OH.HCl$)

4,2 g Lithiumaluminiumhydrid wurden in 150 ml absolutem Tetrahydrofuran suspendiert und im Eisbad auf 0° C gekühlt. Zu dieser Suspension wurden 9,3 g Tritylarginin-

0192135

methylesterhydrochlorid, die in 50 ml absolutem Tetrahydrofuran gelöst waren, langsam zugetropft. Man rührte 30 min bei 0° C und erwärmte dann auf 40-45° C. Nach 30 min wurde das überschüssige Lithiumaluminiumhydrid mit Eiswasser zerstört. Unlösliches wurde durch Filtration entfernt und das Tetrahydrofuran weitgehend abgedampft.

Der Rückstand wurde mit 0,1 N Salzsäure neutralisiert, in n-Butanol/Ethylacetat (1:1/Volumenteile) aufgenommen und mit Wasser gewaschen. Nach Abdampfen des organischen Lösemittels erhielt man Tritylargininolhydrochlorid in der Form eines Schaumes.

| | |
|---|---|
| Ausbeute: | 6,9 g (74,2 %), |
| Reinheitskontrolle: | DC $R_F$ = 0,19 (B) |
| | C/T, Ninhydrin, Sakaguchi und UV positiv |
| | 2,4-Dinitrophenylhydrazin negativ. |

<u>Stufe 3: Argininolditrifluoracetat (Arg $CH_2OH$ . 2 $CF_3COOH$)</u>

1 g Tritylargininolhydrochlorid wurde bei 0° C zu einer Mischung von 80 ml Trifluoressigsäure/Wasser (1:1/Volumenteile) gegeben und 15 min gerührt. Dabei trat eine leichte Gelbfärbung und ein weißer Niederschlag auf. Es wurde über eine Glasfilterfritte abgesaugt und das Filtrat im Vakuum eingeengt. Der Rückstand wurde in Wasser aufgenommen und dreimal mit Ethylacetat extrahiert. Die Wasserphase wurde gefriergetrocknet.

| | |
|---|---|
| Ausbeute: | 950 mg (98 %), |
| Reinheitskontrolle: | DC $R_F$ = 0,1 (A) |
| | C/T, Ninhydrin , Sakaguchi positiv |
| | UV, 2,4-Dinitrophenylhydrazin negativ. |

Stufe 4

870 mg Boc-D-Phe-Pro sowie 370 mg Hydroxybenzotriazol wurden in 10 ml Dimethylformamid gelöst und bei 0° C mit 500 mg Dicyclohexylcarbodiimid versetzt. Nach 30 min wurden 930 mg Argininol x 2 $CF_3COOH$ und 500 µl N-Methylmorpholin zugegeben. Die Reaktionsmischung wurde über Nacht gerührt, vom ausgefallenen Dicyclohexylharnstoff abgefiltert und das Lösemittel im Vakuum abgedampft. Der Rückstand wurde mit 100 ml Ethylacetat verrieben und der dabei entstehende Niederschlag abzentrifugiert. Die Ethylacetat-Lösung wurde abgetrennt und das Lösemittel im Vakuum abgedampft.
Der ölige Rückstand wurde in wenig Ethanol gelöst und das Produkt durch Eintropfen in Diethylether kristallisiert. Die Kristalle wurden abfiltriert und im Hochvakuum getrocknet.

| | |
|---|---|
| Ausbeute: | 1.020 mg (68,7 %), |
| Reinheitskontrolle: | DC $R_F$ = 0,23 (C) |
| | $R_F$ = 0,50 (D) |
| | C/T, UV, Ninhydrin, Sakaguchi positiv |
| | 2,4-Dinitrophenylhydrazin negativ. |

Stufe 5

D-Phe-Pro-Arg-$CH_2OH$ x 2$CF_3COOH$

930 mg Boc-D-Phe-Pro-Arg-$CH_2OH$ x $CF_3COOH$ wurden mit 0,5 ml Anisol und 3 ml eiskalter Trifluoressigsäure 10 min lang gerührt. Die Lösung wurde zur Kristallisation des Produktes in Diethylether getropft. Die Kristalle wurden gesammelt und im Hochvakuum getrocknet.

Ausbeute: 818 mg (86 %),
Reinheitskontrolle: DC RF = 0,31 (A)

Dünnschichtelektrophorese, Pyridinacetatpuffer pH 5,2 auf Celluloseplatten ein Spot
C/T, Ninhydrin, UV, Sakaguchi positiv
2,4-Dinitrophenylhydrazin negativ
Aminosäureanalytik: Phe 1,00, Pro 1,06, Arg 0,04, Gehalt 98 %.

Beispiel 2

Stufe 1: Z-Argininmethylesterhydrochlorid

5,22 g Argininmethylesterdihydrochlorid wurden in Dimethylformamid gelöst und mit 6,83 ml Ethyldiisopropylamin versetzt, wonach 6,8 g Chlorameisensäurebenzylester bei 0° C zugetropft wurden. Die Reaktionsmischung wurde dann drei Stunden bei 40° C und über Nacht bei Raumtemperatur gerührt. Das Lösemittel wurde im Vakuum abgedampft, der Rückstand in Wasser aufgenommen und mit Ethylacetat extrahiert. Die Wasserphase wurde zur Extraktion des Produktes mit n-Butanol/Ethylacetat (1:1/Volumenteile) behandelt. Die organische Phase wurde mit gesättigter Kochsalzlösung ausgewaschen und das Lösemittel wurde im Vakuum abgedampft. Der Rückstand wurde konzentriert in Methanol gelöst und durch Eintropfen in Ethylacetat/Diethylether (1:1/Volumenteile) ausgefällt.

Ausbeute: 5,8 g (81 %),
Reinheitskontrolle: DC $R_F$ = 0,37 (B)
C/T, Sakaguchi, UV positiv
Ninhydrin negativ.

Stufe 2: Z-Argininolhydrochlorid (Z-Arg-$CH_2OH$ x HCl)

5,8 g Z-Argininmethylesterhydrochlorid wurden in 200 ml Ethanol gelöst und 11,8 g Calciumchloriddihydrat zugesetzt. Hierzu gab man 4 g Natriumborhydrid. Anschließend erwärmte man den Ansatz auf 40° C und verfolgte den Verlauf der Reaktion dünnschichtchromatographisch. Es mußte gegebenenfalls noch soviel Natriumborhydrid zugesetzt werden, bis keine Ausgangssubstanz mehr nachweisbar war. Unlösliches wurde daraufhin durch Filtration entfernt und der Rückstand mit Ethanol gewaschen. Das Filtrat wurde mit 30 Volumenprozent Wasser versetzt und der ausgefallene Niederschlag durch Filtration entfernt. Das Lösemittel wurde im Vakuum abgedampft, der Rückstand in Wasser aufgenommen und das Produkt mit Butanol/Ethylacetat (2:1/Volumenteile) durch wiederholtes Ausschütteln extrahiert. Nach Abdampfen des Lösemittels wurde der Rückstand an einer Kieselgelsäule (400 g Kieselgel, Körnung 40-63 µm, Laufmittel E) gereinigt. Die vereinigten Fraktionen wurden durch einen Gelfiltrationsschritt ( Sephadex LH 20, Laufmittel Methanol) weitergereinigt. Nach Abdampfen des Lösemittels erhielt man ein öliges Produkt.

| | |
|---|---|
| Ausbeute: | 4 g (72,6 %), |
| Reinheitskontrolle: | DC $R_F$ = 0,46 (E) |
| | C/T, UV, Sakaguchi positiv |
| | Ninhydrin 2,4-Dinitrophenylhydrazin negativ. |

Stufe 3: H-Argininoldihydrochlorid (H-Arg-$CH_2OH$ x 2 HCl)

2 g Z-Argininolhydrochlorid wurden in Lösemittel Methanol in Gegenwart von Palladium/Aktivkohle hydriert. Der pH-Wert wurde mit 0,1 N Salzsäure auf 4 gehalten. Nach beendeter Wasserstoffaufnahme wurde die Palladium/Aktivkohle durch Filtration entfernt und das Lösemittel im

Vakuum abgedampft.

Ausbeute: 1,36 g (quantitativ),
Reinheitskontrolle: DC $R_F$ = 0,1 (A)
C/T, Ninhydrin, Sakaguchi positiv
2,4-Dinitrophenylhydrazin UV negativ.

Stufe 4

870 mg Boc-D-Phe-Pro sowie 370 mg Hydroxybenzotriazol wurden in 10 ml Dimethylformamid gelöst und bei 0° C mit 500 mg Dicyclohexylcarbodiimid versetzt. Nach 30 min wurden 766 mg Argininol x 2 HCl und 500 µl N-Methylmorpholin zugegeben. Die Reaktionsmischung wurde über Nacht gerührt, vom ausgefallenen Dicyclohexylharnstoff abgefiltert und das Lösemittel im Vakuum abgedampft. Der Rückstand wurde mit 100 ml Ethylacetat verrieben und der dabei entstehende Niederschlag abzentrifugiert. Die Ethylacetat-Lösung wurde abgetrennt und das Lösemittel im Vakuum abgedampft.
Der ölige Rückstand wurde in wenig Ethanol gelöst und das Produkt durch Eintropfen in Diethylether kristallisiert. Die Kristalle wurden abfiltriert und im Hochvakuum getrocknet.

Ausbeute: 1.050 mg (70,72 %),
Reinheitskontrolle: DC $R_F$ = 0,23 (C)
$R_F$ = 0,50 (D)
C/T, UV, Ninhydrin, Sakaguchi positiv
2,4-Dinitrophenylhydrazin negativ.

Stufe 5

D-Phe-Pro-Arg-$CH_2OH$ x $2CF_3COOH$

900 mg Boc-D-Phe-Pro-Arg-$CH_2OH$ x HCl wurden mit 0,5 ml Anisol und 3 ml eiskalter Trifluoressigsäure 10 min lang gerührt. Die Lösung wurde zur Kristallisation des Produktes in Diethylether getropft. Die Kristalle wurden gesammelt und im Hochvakuum getrocknet.

Ausbeute: 789 mg (85 %),
Reinheitskontrolle: DC RF = 0,31 (A)

Dünnschichtelektrophorese, Pyridinacetatpuffer
pH 5,2 auf Celluloseplatten ein Spot
C/T, Ninhydrin, UV, Sakaguchi positiv
2,4-Dinitrophenylhydrazin negativ
Aminosäureanalytik: Phe 1,00, Pro 1,04, Arg 0,03, Gehalt 97 %.

<u>Beispiel 3</u>
<u>Stufe 1/ Boc-Argininol-4-nitrobenzoesäureesterhydrochlorid</u>

Boc-Argininolhydrochlorid wurde durch Reduktion von Boc-Argininmethylesterhydrochlorid analog der Z-Verbindung (Beispiel 2) hergestellt. Die Einführung der Boc-Gruppe erfolgte mit Di-tert. Butylpyrocarbonat (L. Moroder, A. Hallett, E. Wünsch, O. Keller und G. Wersin, Hoppe-Seyler`s; Z. Physiol. Chem. <u>357</u>, 1651-1653 (1976).

500 mg Boc-Arg-$CH_2OH$ wurden in 3 ml Pyridin gelöst bzw. suspendiert und bei 0° C 2 g 4-Nitrobenzoylchlorid portionsweise zugegeben. Man rührte 1 Stunde bei Raumtemperatur und 30 min bei 40° C, wobei eine klare Lösung entstand. Der Ansatz wurde durch Eintropfen in Ether kristallisiert, filtriert und mit Ether nachgewaschen. Der Rückstand wurde in Methanol gelöst, unlösliches

durch Filtration entfernt und mit Ether ausgefällt. Man erhielt ein gelbes Öl, das zur weiteren Reinigung an Kieselgel (100 g 40-63 µm Körnung, Elutionsmittel E) chromatographiert wurde. Die vereinigten Fraktionen wurden nach Abdampfen des Lösemittels an Sephadex LH 20 (Methanol) rechromatographiert.

Ausbeute: 450 mg = (77,6 %)
Reinheitskontrolle: DC $R_F$ = 0.56 (C)

C/T, UV, Ninhydrin, Sakaguchi positiv

Stufe 2: H-Argininol-4-nitrobenzoesäureesterhydrochlorid

430 mg Boc-Argininol-4-nitrobenzoesäureester wurden mit 10 ml 1.2 N HCl/Eisessig unter Feuchtigkeitsanschluß 25 min lang gerührt. Das Abspaltungsreagens wurde im Vakuum abgedampft und zur Vertreibung anhaftender Säurespuren mit Toluol mehrfach abgedampft. Der Rückstand wurde möglichst konzentriert in n-Butanol gelöst und durch Eintropfen in Diethylether kristallisiert.

Die Kristallmasse wurde durch Zentrifugation abgetrennt, mit Ether nachgewaschen und getrocknet.

Ausbeute: 250 mg (67,6 %)
Reinheitskontrolle: DC $R_F$ = 0.44 (A)
UV, C/T, Ninhydrin, Sakaguchi positiv

Stufe 3: Boc-D-Phe-Pro-Argininol-4-nitrobenzoesäureesterhydrochlorid

253 mg Boc-D-Phe-Pro wurden zusammen mit 107 mg Hydroxybenzotriazol in DMF gelöst und bei 0° C mit 154 mg Dicyclohexylcarbodiimid 30 min voraktiviert. Hierzu gab man 240 mg Argininol-4-nitrobenzoesäuredihydrochlorid und 150 µl N-Methylmorpholin. Die Reaktion ließ man über Nacht laufen, dampfte das Lösemittel im Vakuum ab und nahm den öligen Rückstand in n-Butanol/Ethylacetat (1:1, Volumenteile) auf. Die organische Phase wurde dreimal mit Wasser extrahiert und einrotiert. Das Rohprodukt wurde in Butanol konzentriert gelöst und durch Eintropfen in Diethylether kristallisiert. Die Kristalle wurden gesammelt, mit Diethylether nachgewaschen und getrocknet.

Ausbeute: 370 mg (75,6 %)
Reinheitskontrolle: DC $R_F$ = 0.45 (B)
C/T, UV, Ninhydrin, Sakaguchi positiv

Stufe 4: D-Phe-Pro-Argininol-4-nitrobenzoesäureester-dihydrochlorid

350 mg Boc-D-Phe-Pro-Argininol-4-nitrobenzoesäureester-dihydrochlorid wurden zur Abspaltung der Stickstoffschutzgruppe mit 5 ml 1.2 N HCl/Eisessig 25 min lang gerührt. Das Abspaltungsreagens wurde im Vakuum abgedampft und zur Entfernung von Säurespuren zweimal mit Toluol einrotiert. Der ölige Rückstand wurde in Butanol gelöst und durch Eintropfen in Diethylether kristallisiert. Die Kristalle wurden abzentrifugiert, mit Diethylether nachgewaschen und im Hochvakuum getrocknet.

Ausbeute: 290 mg (91,2 %)
Reinheitskontrolle: DC $R_F$ = 0.5 (E)
$R_F$ = 0.43 (A)
C/T, Ninhydrin, UV, Sakaguchi positiv

Beispiel 4: Testung der Inhibitorwirkung gegen α-Humanthrombin

D-Phe-Pro-Argininolditrifluoracetat wurde im Vergleich zu ε-Guanidinocapronsäure-4-carboxyethylphenylester (Foy) getestet.

Testausführung: 700 µl Trispuffer pH 8,2 werden mit 100 µl α-Humanthrombin (0.28 IU) und 100 µl Inhibitorlösung 5 min bei 37° C inkubiert. Nach Zusatz von 100 µl Tos-Gly-Pip-Arg-paranitroanilid (c = 3 mMol/l) wird die Extinktion bei 405 nm gemessen.

Eine 50 %ige Inhibierung wurde im Falle von

D-Phe-ProArgininolditrifluoracetat bei
0.15 ± 0.01 µMol/Liter Endkonzentration erreicht.

Im Falle von

ε-Guanidino-capronsäure-4-carboxyethylphenylester liegt dieser Wert bei
3.65 ± 0.05 µMol/Liter Endkonzentration.

Die Inhibitorwirkung gegen Plasmin bzw. F Xa in analoger Ausführung, wobei die Endkonzentration von D-Phe-Pro-Argininolditrifluoracetat bzw. ε-Guanidino-capronsäure-4-carboxyethylphenylester 0.6 mMol/l ist, ergab folgende Werte:

| | F X a | Plasmin |
|---|---|---|
| D-Phe-Pro-Arg-$CH_2OH$ | 49 % Hemmung | 67 % Hemmung |
| ε-Guanidino-capronsäure-4-carboxyethylphenylester | 91 % Hemmung | 83 % Hemmung |

(Plasminsubstrat: D-Norvalylcyclohexylalanylarginylparanitroanilid (c = 3 mMol/l)

F Xa-Substrat: Bzl-Ile-Glu-Gly-Arg-pNA
(50 % des Glu als Methylester)
(c = 3 mMol/l)

Beim Thrombininhibitionstest (Ausführung siehe oben), zeigte die Substanz Boc-D-Phe-Pro-Arg $CH_2$-OH eine etwa 1 000-fach geringere Wirkung als das H-D-Phe-Pro-Arg $CH_2$-OH.

H-D-Phe-Pro-Argininol-4-nitrobenzoesäureesterdihydrochlorid entsprach in seiner Thrombininhibitionswirkung etwa dem ε-Guanidino-capronsäure-4-carboxyethylphenylester.

Patentansprüche

1. Oligopeptidylargininolderivate und deren Homologe der allgemeinen Formel

X-D-Phe-Pro-A-Y.$(HB)_n$

wobei

| | |
|---|---|
| X | ein Wasserstoffatom oder eine in der Peptidchemie übliche, bekannte Schutzgruppe ist, |
| D-Phe | D-Phenylalanin ist, |
| Pro | L-Prolin ist, |
| A | Argininolrest oder Homologe des Argininols der allgemeinen Formel $-NHCH\left[(CH_2)_mNHC(NH)NH_2\right]CH_2-O-$ wobei m = 2 bis 5 ist, vorzugsweise 3 oder 4; |
| Y | ein Wasserstoffatom oder eine Ester-bildende Gruppe ist, |
| B | ein Säurerest ist |
| n | 0, 1 oder 2 ist. |

2. Verbindungen nach Anspruch 1, dadurch gekennzeichnet, daß A ein L-Argininol-oder L-Homoargininolrest ist.
3. Verbindungen nach Anspruch 1, dadurch gekennzeichnet, daß Y Wasserstoff oder ein Rest mit folgender Struktur darstellt:

Y = -OC-R mit R = H oder einem aliphatischen Rest mit bis zu 4 Kohlenstoffatomen,

R = $-(CH_2)_n-$ 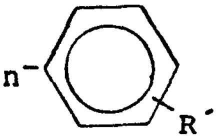 wobei R´= H, $CH_3$, $OCH_3$, $NO_2$, und n = 0, 1 oder 2 ist

Y = $-SO_2-R$ mit R = OH, einem aromatischen oder araliphatischen Rest der Struktur

R = $-(CH_2)_n-$ 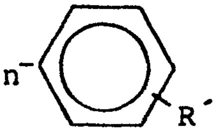 wobei R´= H, $CH_3$, $OCH_3$ und n = 0, 1 oder 2 ist.

4. D-Phenylalanyl-L-prolyl-L-argininol und dessen physiologisch verträglichen Salze.

5. Pharmazeutische Mittel, dadurch gekennzeichnet, daß sie Substanzen gemäß Anspruch 1 oder deren physiologisch verträglichen Salze und einen physiologisch unbedenklichen Träger enthalten.

6. Pharmazeutisches Mittel, dadurch gekennzeichnet, daß sie Substanzen gemäß Anspruch 2 oder deren physiologisch verträglichen Salze und einen physiologisch unbedenklichen Träger enthalten.

7. Pharmazeutisches Mittel enthaltend D-Phenylalanyl-L-prolyl-L-argininol oder dessen physiologisch verträglichen Salze.

8. Verbindung gemäß einem der Ansprüche 1 bis 3 zur Anwendung als Heilmittel.

9. Verbindung gemäß Ansprüchen 1 bis 3 zur Anwendung als Antikoagulans.

10. Verfahren zur Herstellung von Oligopeptidylverbindungen gemäß Anspruch 1, dadurch gekennzeichnet, daß sie durch Fragmentkondensation oder stufenweisen Aufbau

hergestellt werden, wobei temporär eingeführte Schutzgruppen abgespaltet werden und gegebenenfalls die erhaltenen Verbindungen in ihre physiologisch verträglichen Salze überführt werden.

11. Verfahren nach Anspruch 10, dadurch gekennzeichnet, daß Argininole bzw. Homoargininole oder deren Homologe mit reaktiven Säureresten zu Substanzen der Struktur X-A-Y umgesetzt werden, wobei

X ein Wasserstoffatom oder eine in der Peptidchemie übliche Schutzgruppe,

A = Argininol und dessen Homologe und

Y = OC-R mit R = H oder ein aliphatischer Rest mit bis zu 4 Kohlenstoffatomen, oder

R = $-(CH_2)_n-$ 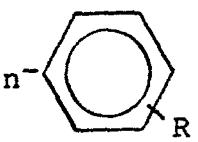 wobei R´= H, $CH_3$, $OCH_3$, $NO_2$ und n = 0, 1 oder 2, sowie

Y = $SO_2$-R mit R = OH, ein aromatischer oder araliphatischer Rest der Struktur

R = $-(CH_2)_n-$ 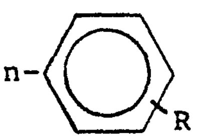 wobei R´= H, $CH_3$, $OCH_3$ und n = 0, 1 oder 2 ist,

die gegebenenfalls vorhandene Schutzgruppe abspaltet und mit einem an der Carboxygruppe aktivierten Derivat einer Verbindung der Formel X-D-Phe-Pro-OH umsetzt und gegebenenfalls die Schutzgruppe abspaltet.

12. Verwendung der Verbindungen gemäß Anspruch 1 bis 3 als Antikoagulantia.